# EUROPEAN PATENT SPECIFICATION

(11) **EP 2 502 632 B1**
(45) Date of publication and mention of the grant of the patent: **28.12.2016**
(21) Application number: 10831468.3
(22) Date of filing: 05.11.2010
(51) Int. Cl.: A61K 38/29, A61P 19/02

(54) **PREVENTATIVE AGENT AND/OR THERAPEUTIC AGENT AND/OR EXACERBATION-SUPPRESSING AGENT FOR HUMAN KNEE OSTEOARTHRITIS**
PRÄVENTIVES MITTEL UND/ODER THERAPIEMITTEL UND/ODER VERSCHLIMMERUNGSUNTERDRÜCKUNGSMITTEL FÜR OSTEOARTHRITIS IM MENSCHLICHEN KNIE
AGENT PROPHYLACTIQUE ET/OU AGENT THÉRAPEUTIQUE ET/OU AGENT DE SUPPRESSION D'EXACERBATION POUR ARTHROSE DE GENOU HUMAIN

(30) Priority: 18.11.2009 US 262214 P
(43) Date of publication of application: 26.09.2012
(73) Proprietor: Asahi Kasei Pharma Corporation, Tokyo 101-8101 (JP)
(72) Inventor: ISHIZUYA Toshinori, Tokyo 101-8101 (JP); KURODA Tatsuhiko, Tokyo 101-8101 (JP)
(74) Representative: Forstmeyer, Dietmar
(86) International application number: PCT/JP2010/069742
(87) International publication number: WO 2011/062073

(56) References cited:
- EP-A1- 2 476 429
- WO-A2-2010/045229
- JP-A- 2007 535 466
- US-A1- 2007 238 769
- US-A1- 2009 010 940
- JE-KEN CHANG ET AL: "Parathyroid hormone 1 34 inhibits terminal differentiation of human articular chondrocytes and osteoarthritis progression in rats", ARTHRITIS & RHEUMATISM, JOHN WILEY & SONS, INC, US, vol. 60, no. 10, 1 October 2009 (2009-10-01), pages 3049-3060, XP008152437, ISSN: 0004-3591, DOI: 10.1002/ART.24843 [retrieved on 2009-09-29]
- T. FUJITA ET AL: "Effect of an Intermittent Weekly Dose of Human Parathyroid Hormone (1-34) on Osteoporosis: A Randomized Double-Masked Prospective Study Using Three Dose Levels", OSTEOPOROSIS INTERNATIONAL, vol. 9, no. 4, 1 April 1999 (1999-04-01), pages 296-306, XP55050510, ISSN: 0937-941X, DOI: 10.1007/s001980050151
- LUGO L ET AL: "089 PTH IMPROVES SYNOVITIS IN AN EXPERIMENTAL MODEL OF OSTEOARTHRITIS PRECEDED BY OSTEOPOROSIS", OSTEOARTHRITIS AND CARTILAGE, BAILLIERE TINDALL, LONDON, GB, vol. 18, 1 October 2010 (2010-10-01), page S47, XP027316645, ISSN: 1063-4584 [retrieved on 2010-09-29]
- CHANG, J. ET AL.: 'Parathyroid hormone 1-34 inhibits terminal differentiation of human articular chondrocytes and osteoarthritis progression in rats' ARTHRITIS AND RHEUMATISM vol. 60, no. 10, 2009, XP008152437
- TAT, S.K. ET AL.: 'Differential modulation of RANKL isoforms by human osteoarthritic subchondral bone osteoblasts: Influence of osteotropic factors' BONE vol. 43, no. 2, 2008, SAN DIEGO, CA, UNITED STATES, pages 284 - 291, XP025671903
- XUE, Y. ET AL.: 'Exogenous PTH-related protein and PTH improve mineral and skeletal status in 25-hydroxyvitamin D-la-hydroxylase and PTH double knockout mice' JOURNAL OF BONE AND MINERAL RESEARCH vol. 20, no. 10, 2005, pages 1766 - 1777, XP008152447

## Description

### Field of the Invention

The present invention relates to a parathyroid hormone (PTH) or a PTH derivative selected from the group consisting of human PTH (1-34) and an acetate salt of human PTH (1-34) for use in preventing and/or treating and/or limiting exacerbation of human knee osteoarthritis.

### Background Art

Knee osteoarthritis is a chronic, degenerative, and proliferative disorder of the joint cartilage, meniscus, ligaments, and other tissues that constitute the joint. The joint pain resulting from knee osteoarthritis and the walking impairment resulting from progressive joint deformation markedly reduce activities of daily living (ADL) and quality of life (QOL) (Non-Patent Document 1). Estimates of the number of knee osteoarthritis patients in Japan as of 2009 range from seven to ten million. When cases are included that are asymptomatic but in which changes characteristic of knee osteoarthritis are radiographically detected, the number reaches thirty million. Establishing methods for preventing the onset of symptoms and for treating knee osteoarthritis is an important sociological issue.

The treatment of knee osteoarthritis comprises conservative therapies and surgical remedies, but treatments for limiting the progression of the disease do not currently exist. Conservative therapies focus on symptomatic treatments for pain, inflammation, and the like. High-tibial osteotomy, artificial joint replacements, and other surgical remedies are applied in cases resistant to conservative therapies, but blood loss, infection, deep-vein thrombosis, pulmonary embolism, abrasion and slackening of the artificial joint, and many other complications and problems have been reported. The current state of knee osteoarthritis treatment is not regarded to be satisfactory (Non-Patent Document 1).

Drug-discovery research for a therapeutic agent that can be expected to limit the progression of the disease has been performed worldwide. A plurality of candidate substances that were found to have medicinal effects in animal models of knee osteoarthritis have actually been evaluated clinically, but most of these trials ended in failure. For example, risedronate was found to have an effect of limiting cartilage degeneration in a spontaneous-onset guinea pig model (Non-Patent Document 2, Non-Patent Document 3). However, medicinal effects were not found in clinical tests on knee osteoarthritis patients (Non-Patent Document 4).

A drug combination of chondroitin and glucosamine was histopathologically found to limit cartilage degeneration in a rat model of anterior cruciate ligament transection (Non-Patent Document 5). However, clinical tests on knee osteoarthritis patients failed to demonstrate pain-relieving activity (Non-Patent Document 6), activity for limiting loss of joint space width (JSW) and limiting disease progression (Non-Patent Document 7), or activity for improving walking distance and WOMAC function (a QOL assessment scale specific to osteoarthritis) (Non-Patent Document 8).

Doxycycline was found to have an effect of preventing disease progression in a dog model of anterior cruciate ligament transection (Non-Patent Document 9). An effect of improving initial-onset osteoarthritis was expected, but clinical tests on knee osteoarthritis patients having mild knee problems corresponding to initial onset failed to demonstrate effects of reducing loss of JSW or reducing pain (Non-Patent Document 10).

Hyaluronate has been reported to have activity for limiting disease progression in a rabbit model of anterior cruciate ligament transection (Non-Patent Document 11) and for accelerating healing in a rabbit model of meniscus damage (Non-Patent Document 12). In Researches using cartilage cells acquired from knee osteoarthritis patients, hyaluronate showed activity for limiting the production of enzymes that degrade the cartilage matrix (Non-Patent Document 13) and activity for limiting apoptosis of cartilage cells (Non-Patent Document 14). However, according to meta-analyses of clinical tests on knee osteoarthritis patients, the efficacy of hyaluronate as a medicine for limiting the progression of the disease is in doubt (Non-Patent Document 15, Non-Patent Document 16).

Estrogen has been reported to have activity for improving cartilage degeneration in mice subjected to orchiectomy (Non-Patent Document 17), in rats subjected to ovariectomy (Non-Patent Document 18), and in monkeys subjected to ovariectomy (Non-Patent Document 19), but according to a systematic review of clinical results, estrogen therapy has not been found to have clear effects but rather has been reported to actually increase the risk of hip osteoarthritis (Non-Patent Document 20).

The use of parathyroid hormone peptide analogues for the treatment of a bone deficit disorder including osteroporosis has been suggested (Patent Document 6). Patent Document 7 dicloses a method for increasing bone mineral density, comprising administering to a mammal en effective amount of a cathepsin K inhibitor and a type of a parathyroid hormone. Further, parathyroid hormone 1-34 has been reported to inhibit terminal differentiation of human articular chondrocytes and osteoarthritis progression in rats (Non-Patent Document 46).

The medicinal effects demonstrated by compounds tested for knee osteoarthritis thus often differ between animal models and clinical studies. In fact, doubts about the validity of animal models as a means of research on osteoarthritis have been raised (Non-Patent Document 21), and the need to establish a gold-standard animal model that is predictive of clinical effects has also been pointed out (Non-Patent Document 22). Effectiveness in humans therefore cannot be predicted from test results in existing animal models, and therefore tests in humans are currently the sole means of evaluation when discovering and identifying agents for preventing and/or treating and/or limiting exacerbation of knee osteoarthritis in humans.

### [Prior Art Documents]

### [Patent Documents]

[Patent Document 1] Japanese Patent No. 3901761
[Patent Document 2] Japanese Patent Application Publication No. 7-238033
[Patent Document 3] Japanese Patent Application Publication No. 2006-137768
[Patent Document 4] Japanese Patent Application Publication No. 64-16799
[Patent Document 5] WO 02/002136
[Patent Document 6] US 2009/0010940 A1
[Patent Document 7] US 2007/0238769 A1

### [Non-Patent Documents]

[Non-Patent Document 1] Hayami, Tadashi. "Osteoarthritis of the knee joint as a cause of musculoskeletal ambulation disability symptom complex (MADS)" Clinical Calcium 18: 32-38, 2008.
[Non-Patent Document 2] Mayer, J et al. "Risedronate but not alendronate slows disease progression in the guinea pig model of primary osteoarthritis (abstract)" J Bone Miner Res 16, Suppl 1: SA472, 2001.
[Non-Patent Document 3] Spector, TD et al. "Potential therapeutic agents for disease modification in osteoarthritis" Aging Clin Exp Res 15: 413-418, 2003.
[Non-Patent Document 4] Bingham, CO et al. "Risedronate decreases biochemical markers of cartilage degeneration but does not decrease symptoms or slow radiographic progression in patients with medial compartment osteoarthritis of the knee" Arthritis Rheum 54: 3494-3507, 2006.
[Non-Patent Document 5] Silva, FS et al. "Combined glucosamine and chondroitin sulfate provides functional and structural benefit in the anterior cruciate ligament transaction model" Clin Rheumatol 28:109-117, 2009.
[Non-Patent Document 6] Clegg, DO et al. "Glucosamine, chondroitin sulfate, and the two in combination for painful knee osteoarthritis" N Engl J Med 354: 795-808, 2006.
[Non-Patent Document 7] Sawitzke, AD et al. "The effect of glucosamine and/or chondroitin sulfate on the progression of knee osteoarthritis." Arthritis Rheum 58: 3183-3191, 2008.
[Non-Patent Document 8] Messier, SP et al. "Glucosamine/chondroitin sulfate combined with exercise for the treatment of knee osteoarthritis: a preliminary study" Osteoarthritis and Cartilage 15: 1256-1266, 2007.
[Non-Patent Document 9] Lucino, PY et al. "Reduction of the severity of canine osteoarthritis by prophylactic treatment with oral doxycycline" Arthritis Rhem 35: 1150-1159, 1992.
[Non-Patent Document 10] Brandt, KD et al. "Effects of doxycycline on progression of osteoarthritis: Result of randomized, placebo-controlled, double-blind trial" Arthritis Rheum 52: 2015-2025, 2005.
[Non-Patent Document 11] Amiel, D et al. "Long-term effect of sodium hyaluronate (Hyalgan) on osteoarthritis progression in rabbit model" Osteoarthritis and Cartilage 11: 636-643, 2003.
[Non-Patent Document 12] Ishima, M et al. "Effects of hyaluronan on the healing of rabbit meniscus injured in the peripheral region" J Orthop Sci 5: 579-584, 2000.
[Non-Patent Document 13] Tanaka, M et al. "Suppressive effects of hyaruronan on MMP-1 and RANTES production from chondrocytes" Rheumatol Int 26: 185-190, 2006.
[Non-Patent Document 14] Lisignoli, G et al. "Anti-Fas-induced apoptosis in chondrocytes reduced by hyaluronan" Arthritis Rheum 44: 1800-1807, 2001.
[Non-Patent Document 15] Lo, GH et al. "Intra-articular hyaluronic acid in treatment of knee osteoarthritis: meta-analysis" JAMA 290: 3115-3121, 2003.
[Non-Patent Document 16] Arrich, J et al. "Intra-articular hyaluronic acid for the treatment of osteoarthritis of the knee: systematic review and meta-analysis" Can Med Assoc J 172: 1039-1043, 2005.
[Non-Patent Document 17] Silberberg, M et al. "Effect of castration and intermittent administration of estrogen on knee joint and femoral shafts of mice" Pathol Microbiol (Basel) 33: 274-286, 1969.
[Non-Patent Document 18] Oestergaard, S et al. "Effects of ovariectomy and estrogen therapy on type II collagen degradation and structural integrity of articular cartilage in rats" Arthritis Rheum 54: 2441-2451, 2006.
[Non-Patent Document 19] Ham, KD et al. "Effects of long-term estrogen replacement therapy on osteoarthritis severity in cynomolgus monkeys" Arthritis Rheum 46: 1956-1964, 2002.
[Non-Patent Document 20] de Klerk, BM et al. "Limited evidence for a protective effect of unopposed oestrogen therapy for osteoarthritis of the hip: a systematic review" Rheumatology 48: 104-112, 2009.
[Non-Patent Document 21] Sasho, Takahisa et al. "Aging and exercise equipment: Basic strategies and goals for osteoarthritis" Journal of the Japan Medical Association 132: 974-976, 2004.
[Non-Patent Document 22] Ameye, LG et al. "Animal models of osteoarthritis: lessons learned while seeking the Holy Grail" Curr Opin Rheumatol 18: 537-547, 2006.
[Non-Patent Document 23] Neer, RM et al. "Effect of parathyroid hormone (1-34) on fractures and bone mineral density in post-menopausal women with osteoporosis" New Engl J Med 344: 1434-1441, 2001.
[Non-Patent Document 24] Sondergaard, BC et al. "PTH has direct anabolic effects on osteoarthritic articular cartilage in vitro and in vivo: is PTH a new treatment for osteoarthritis?" J Bone Miner Res 23: S412, 2008.
[Non-Patent Document 25] Livne, E et al. "Arthicular chondrocytes lose their proliferative activity with aging yet can be restimulated by PTH-(1-84), PGE1, and dexamethasone" J Bone Miner Res 4: 539-548, 1989.
[Non-Patent Document 26] Koike, T et al. "Potent mitogenic effects of parathyroid hormone (PTH) on embryonic chick and rabbit chondrocytes" J Clin Invest 85: 626-632, 1990.
[Non-Patent Document 27] Livne, E et al. "In vitro effect of hormone and growth factors on the incorporation of [3H] leucine, [35S] sulfate, and [3H] proline by chrondrocytes of aging mice" Mech Aging Dev 72: 213-229, 1993.
[Non-Patent Document 28] Kitamura H et al. "Establishment of a bipotent cell line CL-1 which differentiates into chondrocytes and adipocytes from adult mouse" Osteoarthritis and Cartilage 12: 25-37, 2004.
[Non-Patent Document 29] Ishizuya, Toshinori et al. "Activity of parathyroid hormone on bone tissue in in vitro tests" Journal of Japanese Society of Bone Morphometry (Nichi Hokkei Zasshi) 5: 1-5, 1995.
[Non-Patent Document 30] Yamamoto, Michiko. "Physiological activity of parathyroid hormone-related peptide" Japanese Journal of Clinical Medicine 63 (Clinical Molecular Endocrinology 3): 377-381, 2005.
[Non-Patent Document 31] Kohno, H et al. "Synovial fluids from patients with osteoarthritis and rheumatoid arthritis contain high levels of parathyroid hormone-related peptide" J Bone Miner Res 12: 847-854, 1997.
[Non-Patent Document 32] Terkeltaub, R et al. "Parathyroid hormone-related protein is abundant in osteoarthritic cartilage, and the parathyroid hormone-related protein 1-173 isoform is selectively induced by transforming growth factor beta in articular chondrocytes and suppresses generation of extracellular inorganic pyrophosphate" Arthritis Rheum 41: 2152-2164, 1998.
[Non-Patent Document 33] Nakase, T et al. "Localization of bone morphogenetic protein-2 in human osteoarthritic cartilage and osteophytes" Osteoarthritis and Cartilage 11: 278-284, 2003.
[Non-Patent Document 34] Scharstuhl, A et al. "Reduction of osteophyte formation and synovial thickening by adenoviral overexpression of transforming growth factor ß/bone morphogenetic protein inhibitors during experimental osteoarthritis" Arthritis Rheum 48: 3442-3451, 2003.
[Non-Patent Document 35] Davidson, ENB et al. "Resemblance of osteophytes in experimental osteoarthritis to transforming growth factor 6-induced osteophytes" Arthritis Rheum 56: 4065-4073, 2007.
[Non-Patent Document 36] Massicotte, F et al. "Modulation of insulin-like growth factor levels in human osteoarthritic subchondral bone osteoblasts" Bone 38: 333-341, 2006.
[Non-Patent Document 37] Uchio, Yuji. "Definition of knee OA" in Guidebook of Conservative Medical Management of Knee Osteoarthritis (Iwaya Tsutomu, ed.). Medical Review, Co. Tokyo, 2005. p. 16-26.
[Non-Patent Document 38] Brandt, KD et al. "Workshop on etiopathogenesis of osteoarthritis" J Rhematol 13: 1126-1160, 1986.
[Non-Patent Document 39] Keuttner, K et al. "Osteoarthritic disorders" American Academy of Orthopaedic Surgeons, Rosement, PA USA, 1995. p. 21-25.
[Non-Patent Document 40] Altman, R et al. "Development of criteria for the classification and reporting of osteoarthritis" Arthritis Rheum 29: 1039-1049, 1986.
[Non-Patent Document 41] "Definition and concept of osteoporosis" in Guidelines for the Treatment and Prevention of Osteoporosis, 2006 edition (edited by the Committee for Creating Guidelines for the Treatment and Prevention of Osteoporosis). Life Sciences Publishing, 2006. p. 2-3.
[Non-Patent Document 42] Orimo, Hajime et al. "Diagnostic criteria for primary osteoporosis (1996 revised edition)" Japan Journal of Bone Metabolism 14: 219-233, 1997.
[Non-Patent Document 43] Takai, M et al. Peptide Chemistry 1979: 187-192, 1980.
[Non-Patent Document 44] Marcus, R et al. "Bioassay of parathyroid hormone in vitro with a stable preparation of adenyl cyclase from rat kidney" Endocrinology 85: 801-810, 1969.
[Non-Patent Document 45] Kojima, Toshihisa et al. "III. Evaluating joint cartilage degeneration in OA. 3. Clinical pathology of knee osteoarthritis" The Bone 23: 51-54, 2009.
[Non-Patent Document 46] Je-Ken Chang et al. "Parathyroid hormone 1-34 inhibits terminal differentiation of human articular chondrocytes and osteoarthritis progression in rats" Arthritis Rheum 60: 3049-3060, 2009.

### SUMMARY OF THE INVENTION

### Problem to Be Solved by the Invention

It is an object of the present invention to identify and provide a therapeutic agent that is extremely useful for preventing and/or treating and/or limiting exacerbation of human knee osteoarthritis.

### Means to Solve the Problem

As a result of the thorough-going research efforts of the present inventors, it was discovered that the symptoms of knee osteoarthritis are markedly limited when parathyroid hormone (PTH) or a PTH derivative is administered to humans once per week.

As therapeutics for osteoporosis, PTH and PTH derivatives have been proven to have activities for increasing bone density of the lumbar spine and femoral neck and for limiting vertebral and non-vertebral fractures (Non-Patent Document 23). However, there have been no reports showing that PTH or PTH derivatives have effects of preventing, treating, or limiting exacerbation of knee osteoarthritis in humans. More specifically, conventionally, a slight therapeutic effect was reported for PTH using a rat model of osteoarthritis (Non-Patent Document 24), but, as discussed in the Background Art section, the fact that efficacy in humans cannot be predicted from test results in animal models was also shown at the same time (Non-Patent Documents 21, 22, et al.). Ultimately, absolutely no data showing effects of preventing or limiting exacerbation of human knee osteoarthritis, and certainly no effects of treating the disease, have been shown for PTH or PTH derivatives in any prior art documents. Suggestions of the present invention are therefore also absent in the prior art.

Activity of PTH and PTH derivatives on cultured cartilage cells and tissue has been reported (Non-Patent Documents 25 through 28), but none of the cases went beyond observations on the effects on cultured cells. Besides the fact that these results are not regarded to qualify as a description of effects within the human body, it is well-known that the action of PTH and PTH derivatives on cultured cells and tissue is greatly affected by the species of animal that is the source of the cells or tissue, the cell type, the cell density, the stage of differentiation of the cells, and the stimulus time (Non-Patent Document 29), such that even the conclusions and predictions drawn from the actual results in these documents differ. Accordingly, at best, the observations of culture experiments obviously cannot provide a shortcut to predictions of efficacy in the human body.

Parathyroid-hormone-related peptide (PTHrP) was described as a therapeutic agent for osteoarthritis and inflammatory arthritic disorders in Patent Document 1, but, to begin with, PTHrP is not only a substance that has a completely different chemical structure from PTH and PTH derivatives, but the pharmacological activity is also different from PTH and PTH derivatives, which primarily act to regulate calcium metabolism, and is primarily related to interactions between epidermal cells and mesenchymal cells, e.g., the eruption of teeth, and histogenesis of the papillae and mammary glands (Non-Patent Document 30). Due to the fact that high concentrations of PTHrP have been found in the joint fluid of knee osteoarthritis patients, the possibility has also been raised that PTHrP is in fact an exacerbating factor in knee osteoarthritis (Non-Patent Documents 31 and 32). The description in the aforedescribed Patent Document 1 therefore also does not allow PTH or PTH derivatives to be predicted to have effects of preventing and/or treating and/or limiting exacerbation of knee osteoarthritis in humans.

Patent Document 2 and Patent Document 3 describe techniques for using PTH and PTH derivatives as accelerating agents in surgical chondroplasty. However, whereas in these techniques PTH and PTH derivatives are used in an auxiliary fashion during periods limited to operations for performing surgical curettage of joint cartilage in order to reach the subchondral bone, the present invention has as a target knee osteoarthritis characterized by degeneration of the cartilage and is related to an agent for preventing and/or treating and/or limiting exacerbation of that disease. The mechanism of action is therefore completely different between the two approaches. As discussed above in the prior art, effectiveness in humans also cannot be predicted from the test results in the animal models described as test examples in Patent Document 2 and Patent Document 3, and the existing knowledge in the aforedescribed prior art documents therefore does not allow PTH and PTH derivatives to be predicted to have effects of preventing and/or treating and/or limiting exacerbation of knee osteoarthritis in humans.

Finally, a particularly important point is that the formation of osteophytes on, e.g., the neighboring parts of subchondral bone accompanies the progression of disease in knee osteoarthritis. The osteophytes themselves or loose bodies thereof restrict the range of activity and are a large factor in reducing ADL and QOL of the patient. However, it has been reported that bone morphogenetic factor-2 (BMP-2) and transforming growth factor-ß (TGF-ß) are involved in osteophyte formation (Non-Patent Documents 33 through 35). BMP-2 and TGF-ß are known osteogenesis-stimulating factors. It is therefore thought that a person having ordinary skill in the art would readily and unambiguously presume a danger that administering PTH and PTH derivatives, which have similar osteogenesis-stimulating activity, would actually stimulate the onset of knee osteoarthritis and/or exacerbate knee osteoarthritis. In fact, the amount of insulin-like growth factor-I (IGF-I) produced in osteoblasts from the subchondral bone of knee osteoarthritis patients is positively correlated with disease progression, and the possibility that PTH may function as one of the biological factors that facilitates production of IGF-I has been raised (Non-Patent Document 36).

In contrast to the above prior art, the present inventors, upon employing clinical tests for thorough-going investigations into the effects of PTH and PTH derivatives on human knee osteoarthritis, discovered that these compounds have extremely exceptional effects of preventing and/or treating and/or limiting exacerbation of human knee osteoarthritis, and arrived at the present invention.

In other words, the clinical effects of PTH and PTH derivatives on knee osteoarthritis that have been made clear herein for the first time by the present inventors were completely unknown until the present. Simply put, the reports of the prior art do not exceed tests using animal models and cultured cells and cannot be used to predict these effects. Parts of the prior art present inconsistent results and discussions between reports, and since the PTH, PTH derivatives, and the like have actually been suggested to have a role as exacerbating factors in knee osteoarthritis, the newly discovered solid evidence in humans, which evidence resulted from clinical tests performed by the present inventors, is a breakthrough discovery that is extremely useful for the advancement of medical technology.

As active ingredients in a therapeutic agent for osteoporosis, the PTH and the PTH derivatives also have exceptional activity for increasing bone density in the lumbar spine and femoral neck and for limiting vertebral and non-vertebral fractures, as described above (Non-Patent Document 23) and are therefore particularly advantageous as medicines for patients in which osteoporosis and knee osteoarthritis are coincident. The discovery of such a medicine is extremely compatible with related medical goals, is linked to lessening the burden on patients and healthcare providers, and is also extremely meaningful from the standpoint of healthcare economics.

Therefore, the present invention relates to a parathyroid hormone (PTH) or a PTH derivative selected from the group consisting of human PTH (1-34) and an acetate salt of human PTH (1-34) for use in preventing and/or treating and/or limiting exacerbation of human knee osteoarthritis.

In a preferred aspect of the invention, the PTH for use in preventing and/or treating and/or limiting exacerbation of human knee osteoarthritis is human PTH (1-84).

In another preferred aspect of the invention, the PTH derivative for use in preventing and/or treating and/or limiting exacerbation of human knee osteoarthritis is human PTH (1-34) or an acetate salt of human PTH (1-34).

The present invention further relates to a PTH or a PTH derivative selected from the group consisting of human PTH (1-34) and an acetate salt of human PTH (1-34) for use in preventing and/or treating and/or limiting exacerbation of human knee osteoarthritis concomitant with osteoporosis.

In the same manner, in a preferred aspect of the invention, the PTH for use in preventing and/or treating and/or limiting exacerbation of human knee osteoarthritis concomitant with osteoporosis is human PTH (1-84).

In another preferred aspect of the invention, the PTH derivative for use in preventing and/or treating and/or limiting exacerbation of human knee osteoarthritis concomitant with osteoporosis is human PTH (1-34) or an acetate salt of human PTH (1-34).

The present invention also relates to a medicine comprising parathyroid hormone (PTH) or a PTH derivative selected from the group consisting of human PTH (1-34) and an acetate salt of human PTH (1-34) as an active ingredient for use in preventing and/or treating and/or limiting exacerbation of human knee osteoarthritis; and especially for use in preventing and/or treating and/or limiting exacerbation of human knee osteoarthritis concomitant with osteoporosis.

### Effects of the Invention

Parathyroid hormone (PTH) or a PTH derivative selected from the group consisting of human PTH (1-34) and an acetate salt of human PTH (1-34) can extremely effectively prevent and/or treat and/or limit exacerbation of knee osteoarthritis in humans, and in particular exacerbation of human knee osteoarthritis concomitant with osteoporosis. The medicine of the present invention, which is characterized in comprising a parathyroid hormone (PTH) or a PTH derivative selected from the group consisting of human PTH (1-34) and an acetate salt of human PTH (1-34) as an active ingredient for use in preventing and/or treating and/or limiting exacerbation of human knee osteoarthritis, can extremely effectively prevent and/or treat and/or limit exacerbation of knee osteoarthritis in humans. The medicine of the present invention can also prevent and/or treat and/or limit exacerbation of human knee osteoarthritis concomitant with osteoporosis.

### DESCRIPTION OF THE PREFERRED EMBODIMENTS

The "active ingredient" in the medicine of the present invention is designated as a parathyroid hormone (PTH) or a PTH derivative selected from the group consisting of human PTH (1-34) and an acetate salt of human PTH (1-34).

The PTH in the present invention may be any compound that is at least a peptide hormone that has activity for increasing the concentration of calcium in the blood within the body, and includes naturally occurring PTH, PTH manufactured using genetic engineering methods, and chemically synthesized PTH. The PTH preferably comprises the human PTH that is composed of 84 amino acids (human PTH (1-84)).

The PTH derivatives are selected from the group consisting of human PTH (1-34) and an acetate salt of human PTH (1-34).

In particular, PTH (1-34) corresponds to a parathyroid-hormone fragment composed of 34 amino acids from the N-terminal to amino acid 34 of parathyroid hormone. The biological activity of naturally occurring PTH is known to be replicated by PTH (1-34) (Biochemistry Dictionary, Tokyo Kagaku Dojin, 1984), which is used clinically as a diagnostic agent for parathyroid function and as a therapeutic agent for osteoporosis.

The aforedescribed PTH or PTH derivative has extremely low toxicity. Fatal toxicity is never observed, even when, e.g., 3300 or 2000 units/kg (body weight) of human PTH (1-34) is administered to mice and rats by intravenous, intramuscular, subdermal, or oral routes.

The medicine of the present invention is useful for preventing and/or treating and/or limiting exacerbation of human knee osteoarthritis. The medicine of the present invention is also useful for preventing and/or treating and/or limiting exacerbation of human knee osteoarthritis concomitant with osteoporosis.

The term "osteoarthritis" used in the present specification is defined as "a disorder occurring as a result of regressive degeneration of joint cartilage and joint constituents, as well as the subsequent destruction and proliferative changes of cartilage and bone." In particular, "knee osteoarthritis" is defined as "a disorder resulting from occurrence of the aforementioned changes in a knee joint, as a result of which signs and symptoms are presented in the knee" (Non-Patent Document 37). For more detailed definitions, see Non-Patent Documents 38 and 39.

From the perspective of routine medical care, a definition of osteoarthritis for which prevention and/or treatment and/or exacerbation limitation should be instituted using the medicine of the present invention can also be given in the context of diagnostic criteria for osteoarthritis or knee osteoarthritis. The osteoarthritis or knee osteoarthritis diagnostic criteria that are used may be, e.g., publicly proposed guidelines or the criteria set by medical institutions, but diagnostic criteria that are widely used throughout the world are the knee osteoarthritis classification criteria proposed by the American Rheumatism Association in 1986 (Non-Patent Document 40). Knee osteoarthritis for which prevention and/or treatment and/or exacerbation limitation should be instituted using the medicine of the present invention can be defined with reference to these criteria.

The medicine of the present invention is used to prevent and/or treat and/or limit the exacerbation of diseases defined as knee osteoarthritis as described above. That is, the medicine of the present invention can be used for the above purpose insofar as the disease conditions of the subject fall within the knee osteoarthritis defined as above.

The term "osteoporosis" used in the present specification designates the disorder defined by a Consensus Development Conference of the American National Institutes of Health (NIH) in 2000 as "a skeletal disorder characterized by compromised bone strength predisposing to an increased risk of fracture" (Non-Patent Document 41). From the perspective of routine medical care, a definition of osteoporosis concomitant with knee osteoarthritis for which prevention and/or treatment and/or exacerbation limitation should be instituted using the medicine of the present invention can also be given in the context of diagnostic criteria for osteoporosis. The osteoporosis diagnostic criteria that are used may be, e.g., publicly proposed guidelines or the criteria set by medical institutions, but reference may also be made to the diagnostic criteria proposed by the Japanese Society for Bone and Mineral Research in 1997 (Non-Patent Document 42).

Osteoporosis is generally classified by type into primary osteoporosis, when no underlying disorder is present, and secondary osteoporosis, when accompanying any of various endocrine orders, blood disorders, or other disorders. However, the knee osteoarthritis that is prevented and/or treated and/or limited in exacerbation by the medicine of the present invention may be concomitant with osteoporosis of any type, without limitation due to, e.g., the type of osteoporosis, the degree of progression, or the presence or absence of existing fractures. However, it should be noted that even in cases where knee osteoarthritis and osteoporosis are coincident, the disease defined as knee osteoarthritis in the present invention and the disease defined as osteoporosis are not necessarily the same, and, furthermore, patients to receive treatment for preventing, treating, or limiting exacerbation of knee osteoarthritis are not necessarily the same as patients to receive treatment for osteoporosis. In other words, accompanying loss of bone mass is the general rule in osteoporosis, but in contrast, in knee osteoarthritis the bones frequently exhibit proliferation and hardening in order to compensate for the wearing away of joint cartilage. Dequecker et al. point out that osteoporosis is common in ectomorphs, whereas knee osteoarthritis is common in endomorphs, and that from a clinical perspective, as well, patients developing only one or the other of these disorders are not uncommon (see Dequecker, J, Goris, P. "Osteoarthritis and osteoporosis" JAMA 249: 1448-1451, 1983).

The medicine of the present invention is therefore preferably used for preventing and/or treating and/or limiting exacerbation of human knee osteoarthritis, and the medicine of the present invention is even more preferably used for preventing and/or treating and/or limiting exacerbation of human knee osteoarthritis concomitant with osteoporosis.

Auxiliary ingredients are preferably added to the PTH or PTH derivative, which is the active ingredient, as necessary and as permitted pharmaceutically in the manufacture of the medicine of the present invention, creating a medicinal composition. However, the selection of the auxiliary ingredients and the mixture with the active ingredient are preferably properly adjusted so as to avoid interactions that substantially reduce the pharmaceutical efficacy of the PTH or the PTH derivative under typical usage conditions. It shall also be apparent that the pharmaceutically permissible auxiliary ingredients need to be provided with both adequately high purity and adequately low toxicity to ensure that no safety problems are presented upon administration to humans. Examples of pharmaceutically permissible auxiliary ingredients include sugars, starch, cellulose derivatives, gelatin, stearic acid, magnesium stearate, vegetable oils, polyols, alginic acid, tonicity agents, buffering agents, wetting agents, lubricants, colorants, odorants, preservatives, stabilizing agents, antioxidants, antiseptic agents, and anti-microbial agents.

Examples of the medicinal form of the medicine of the present invention include injected agents, rectally absorbed agents, vaginally absorbed agents, nasally absorbed agents, transdermally absorbed agents, transpulmonarily absorbed agents, intraorally absorbed agents, and orally administered agents. These specific forms of administration are not given by way of limitation.

When administering the medicine of the present invention as, e.g., an injected agent, the medicine is preferably adjusted for intramuscular, subdermal, or intravenous administration. When administering the medicine as a rectally or vaginally absorbed agent, the form is generally that of a suppository. When administering the medicine as a nasally or transdermally absorbed agent, the pharmaceutical form may involve the addition of appropriate absorbefacients. When administering the medicine as a transpulmonarily absorbed agent, the form may be that of an aerosol composition containing water or an appropriate dispersant, as well as a propellant. When administering the medicine as an intraorally absorbed agent, the form of, e.g., a sublingual tablet to which appropriate absorbefacients have been added may be used. When administering the medicine as an orally administered agent, the form may be that of a liposome formulation, a microcapsule formulation, or other oral format.

As a more specific example of the medicine of the present invention when formulated as an injected agent; e.g., human PTH (1-34) is dissolved into distilled water for injection, in which appropriate amounts of buffering agents, tonicity agents, and pH-regulating agents have been dissolved, and the solution is then passed through a sterilization filter. The sterilized solution is dispensed into ampoules, whereby the desired injected agent can be prepared.

When the medicine of the present invention is formulated as a rectally or vaginally absorbed agent; e.g., human PTH (1-34) is dissolved or dispersed into distilled water or an oil-based solvent along with appropriately selected absorbefacients having chelating abilities (e.g., sodium pectate, sodium alginate) and hypertonicity agents (e.g., sodium chloride, glucose), and an anally or vaginally injected agent or a suppository can be created (see the specifications of British Patent Nos. 2092002 and 2095994).

When the medicine of the present invention is formulated as a nasally absorbed agent; e.g., a liquid or powdered formulation can be created in which glucuronic acid, succinic acid, tartaric acid (water-soluble organic acids), or another absorbefacient is added to human PTH (1-34) (Japanese Patent Application Publication Nos. 63-243033, 63-316737, 1-230530, 2-111, 2-104531). A nasally absorbed agent can also be obtained from, e.g., adding human PTH (1-34) to an appropriate emulsion (Japanese Patent Application Publication No. 4-99729).

When the medicine of the present invention is formulated as a transdermally absorbed agent; e.g., methods for adding Azone or another absorbefacient and promoting absorption of the active ingredient from the skin (Second Annual Meeting of the Academy of Pharmaceutical Science and Technology, Japan, Collected Presentation Summaries p. 57-58), and methods employing iontophoresis (Ann NY Acad Sci 507: 32, 1998) have been reported. The medicine of the present invention may therefore be prepared with reference to these techniques.

When the medicine of the present invention is formulated as an agent absorbed transpulmonarily methods have been disclosed in which, e.g., the active ingredient and Arlacel, Span 80, or another dispersant are pulverized and levigated, and a homogenous paste is obtained, after which the paste is dispersed into cooled Freon 11, Freon 12, or another propellant, and thereafter packed into a container provided with a valve (Japanese Patent Application Publication No. 60-161924). The medicine of the present invention may therefore be prepared with reference to these techniques.

When the medicine of the present invention is formulated as an intraorally absorbed agent, methods have been disclosed for, e.g., adding ascorbic acids, acidic amino acids, citric acids, or unsaturated fatty acids, alone or in a combination of two or more; glucose or another excipient; menthol or another flavoring agent; and the like to the active ingredient and preparing a troche, sublingual tablet, powdered agent, or the like (Japanese Patent Application Publication No. 56-140924). The medicine of the present invention may therefore be prepared with reference to these techniques.

When the medicine of the present invention is formulated as an orally administered agent, methods have been disclosed for, e.g., preparing orally administered agents from PTH or PTH derivatives (Pharm Res 18: 964-970, 2001). The medicine of the present invention may therefore be prepared with reference to these techniques.

The dosage of the PTH or PTH derivative included as the active ingredient of the medicine of the present invention differs according to age, body type, sex, health of the subject, specific activity of the administered PTH or PTH derivative, medicinal form, and frequency of administration, among other parameters, but using as a standard, e.g., an effective does of human PTH (1-34) subdermally administered for treating osteoporosis, the dosage is 10 to 2000 units/person/day (week), preferably 20 to 1000 units/person/day (week), more preferably 60 to 700 units/person/day (week), and even more preferably 100 to 200 units/person/day (week). When effective dosages are administered using the corresponding weights as a standard, the effective dosage (units) of the given PTH or PTH derivative can be converted to the effective dosage of the weight standard using the activity-measuring method described in an article by Marcus et al. (Non-Patent Document 44). Using as an example the effective dosage of a weight standard of subdermally administered human PTH (1-34), approximately 3 to 560 µg/person/day (week), preferably approximately 6 to 280 µg/person/day (week), more preferably approximately 20 to 212 µg/person/day (week), and even more preferably approximately 30 to 60.6 µg/person/day (week) is administered to the subject. Appropriate adjustments may be made with reference to the ranges of these usage amounts according to the state of the subject and the form of the medicine.

The frequency of administering the PTH or PTH derivative included as the active ingredient of the medicine of the present invention differs according to age, body type, sex, health of the subject, specific activity of the administered PTH or PTH derivative, dosage, and medicinal form, among other parameters, but may range from once per month to three time per day, preferably once per week to once per day, and more preferably once per week or once per day.

The medicine of the present invention does not interact with other agents and can therefore be combined with a variety of medicinal agents according to the condition of the subject. There is no limitation on the medicinal agents for possible combination with the medicine of the present invention.

### [Examples]

Working examples and test examples are given below and the present invention described in detail, but the present invention is not in any way limited thereby.

### [Example 1]

### [Test Example 1] Effects of human PTH (1-34) on knee osteoarthritis concomitant with osteoporosis (1).

Female patients diagnosed with primary osteoporosis on the basis of the diagnostic criteria of Non-Patent Document 42 were given intermittent subdermal administrations of 5 or 100 units of an acetate salt of human PTH (1-34), which was prepared using the method of Takai (Patent Documents 4 and 5, Non-Patent Document 43), once per week (accordingly, there was a 5-unit administration group and a 100-unit administration group). The measured activity of the acetate salt of human PTH (1-34) was 3300 units/mg in the present test example according to the article of Marcus et al. (Non-Patent Document 44).

An injectable formulation containing 5 or 100 units per vial of the acetate salt of human PTH (1-34) was dissolved into 1 mL of normal saline solution at the time of usage, and 1 mL/person was subdermally administered to the 5-unit and 100-unit groups. Two tablets of a calcium agent (containing 500 mg of precipitated calcium carbonate (200 mg of calcium) per tablet) were further administered orally once per day to the 5-unit and 100-unit groups.

During the course of administration, both groups were prohibited from concurrent usage of calcitonin formulations, activated vitamin D3 formulations, vitamin K formulations, ipriflavone formulations, formulations of salts of bisphosphonic acid, estrogen formulations, anabolic-hormone formulations, doctor-prescribed calcium formulations (excepting the aforedescribed calcium agent administered as two tablets per day), and other medicinal agents thought to exert an effect on bone metabolism.

The attending physicians evaluated knee osteoarthritis according to the diagnostic criteria of each medical treatment facility on the basis of, e.g., the chief complaints of the patient, such as knee-joint pain and knee pain.

The ages and the average administration periods of the human PTH (1-34) of the two groups are as shown in Table 1. No statistically significant difference was found between the backgrounds of the two groups.

[Table 1]

**Table 1: Patient backgrounds of 5- and 100-unit administration groups**

| | Number of cases | Age (years; average ± standard deviation) | Average administration period of human PTH (1-34) (days) |
|---|---|---|---|
| 5-unit administration group | 165 | 71.2 ± 7.2 | 330.1 ± 241.3 |
| 100-unit administration group | 314 | 70.2 ± 8.1 | 295.2 ± 190.7 |

The number of cases newly recognized as knee osteoarthritis and the frequency of onset during the administration period are shown in Table 2. The frequency of onset of knee osteoarthritis in the 100-unit administration group was significantly less than that in the 5-unit administration group (p < 0.05).

[Table 2]

**Table 2: Onset of knee osteoarthritis**

| | Number of cases of onset of knee osteoarthritis | Frequency of onset (%) |
|---|---|---|
| 5-unit administration group | 11 | 6.67 |
| 100-unit administration group | 4 | 1.27* |

| | | |
|---|---|---|
| * Difference with 5-unit administration group: p < 0.05 | | |

The frequency of onset of knee osteoarthritis in the 5-unit administration group in the present test example was also compared to the natural frequency of onset. The value used for the natural frequency of onset resulted from dividing the 174,000 female arthritis patients of ages 70 through 74 throughout the country in the Japanese Nationwide Patient Survey (2005, edited by the Statistics and Information Department of the Minister's Secretariat of the Ministry of Health, Labour and Welfare, [published by] the Health and Welfare Statistics Association) by the population of 3,597,754 women of the same age throughout the country according to the Statistics Bureau of the Ministry of Internal Affairs and Communications.

The natural frequency of onset of knee osteoarthritis calculated from the above is 4.84%, and a statistically significant difference was not found with the 6.67% frequency of onset in the 5-unit administration group. According to the above, the frequency of onset of knee osteoarthritis in the 5-unit administration group was assumed to be equal to natural onset, and the 100-unit administration group was thought to have significantly more limited onset of knee osteoarthritis than in the case of natural onset.

### [Test Example 2] Effects of human PTH (1-34) on knee osteoarthritis concomitant with osteoporosis (2).

Female patients diagnosed with primary osteoporosis on the basis of the diagnostic criteria of Non-Patent Document 42 were given intermittent subdermal administrations of normal saline solution and 200 units of an acetate salt of human PTH (1-34), which was prepared using the method of Takai (Patent Documents 4 and 5, Non-Patent Document 43), once per week (accordingly, there was a placebo group and a 200-unit administration group).

Two tablets of a calcium agent (containing 500 mg of precipitated calcium carbonate (200 mg of calcium) per tablet) were further administered orally once per day to the placebo group and the 200-unit administration group.

During the course of administration, both groups were prohibited from concurrent usage of calcitonin formulations, activated vitamin D3 formulations, vitamin K formulations, ipriflavone formulations, formulations of salts of bisphosphonic acid, estrogen formulations, anabolic-hormone formulations, doctor-prescribed calcium formulations (excepting the aforedescribed calcium agent administered as two tablets per day), and other medicinal agents thought to exert an effect on bone metabolism.

The attending physicians evaluated knee osteoarthritis according to the diagnostic criteria of each medical treatment facility on the basis of, e.g., the chief complaints of the patient, such as knee-joint pain and knee pain.

The ages and average administration periods of both groups are as shown in Table 3. No statistically significant difference was found between the backgrounds of the two groups.

[Table 3]

**Table 3: Patient backgrounds of placebo and 200-unit administration groups**

| | Number of cases | Age (years; average ± standard deviation) | Average administration period (weeks) |
|---|---|---|---|
| Placebo group | 278 | 75.4 ± 5.8 | 65.5 ±17.0 |
| 200-unit administration group | 277 | 75.1 ±5.9 | 55.7 ± 26.4 |

The number of cases newly recognized as knee osteoarthritis, the number of cases of knee osteoarthritis found to be exacerbated, and the respective frequencies thereof during the administration period are shown in Table 4. The frequency of onset of knee osteoarthritis in the 200-unit administration group was less than that in the placebo group. The effect of the medicine of the present invention is more pronounced in patients less than 75 years of age, who are assumed to be comparatively active.

[Table 4]

**Table 4: Onset and exacerbation of knee osteoarthritis**

| | Onset of knee osteoarthritis | | Exacerbation of knee osteoarthritis | |
|---|---|---|---|---|
| | Number of cases of onset | Frequency of onset (%) | Number of cases of exacerbation | Frequency of exacerbation (%) |
| Placebo group | 17 | 6.1 | 7 | 2.5 |
| 100-unit administration group | 15 | 5.4 | 4 | 1.4 |

According the present test example, administration of human PTH (1-34) to osteoporosis patients clearly and markedly limits the onset and exacerbation of knee osteoarthritis, and the effectiveness of human PTH (1-34) for treating and/or limiting exacerbation of knee osteoarthritis is manifest.

### [Industrial Applicability]

The medicine of the present invention can be appropriately used in fields for preventing and/or treating and/or limiting exacerbation of human knee osteoarthritis.

## Claims

1. A parathyroid hormone (PTH), or a PTH derivative selected from the group consisting of human PTH (1-34) and an acetate salt of human PTH (1-34), for use in preventing and/or treating and/or limiting exacerbation of human knee osteoarthritis.

2. The PTH or PTH derivative for use as in claim 1, wherein the PTH is human PTH(1-84).

3. The PTH or PTH derivative for use as in claim 1, wherein the PTH derivative is human PTH(1-34).

4. The PTH or PTH derivative for use as in claim 1, wherein the PTH derivative is an acetate salt of human PTH(1-34).

5. A parathyroid hormone (PTH), or a PTH derivative selected from the group consisting of human PTH (1-34) and an acetate salt of human PTH (1-34), for use in preventing and/or treating and/or limiting exacerbation of human knee osteoarthritis concomitant with osteoporosis.

6. The PTH or PTH derivative for use as in claim 5, wherein the PTH is human PTH(1-84).

7. The PTH or PTH derivative for use as in claim 5, wherein the PTH derivative is human PTH(1-34).

8. The PTH or PTH derivative for use as in claim 5, wherein the PTH derivative is an acetate salt of human PTH(1-34).

9. A medicine comprising parathyroid hormone (PTH), or a PTH derivative selected from the group consisting of human PTH (1-34) and an acetate salt of human PTH (1-34), as an active ingredient for use in preventing and/or treating and/or limiting exacerbation of human knee osteoarthritis.

10. A medicine comprising parathyroid hormone (PTH), or a PTH derivative selected from the group consisting of human PTH (1-34) and an acetate salt of human PTH (1-34), as an active ingredient for use in preventing and/or treating and/or limiting exacerbation of human knee osteoarthritis concomitant with osteoporosis.

11. The medicine for use according to claim 9 or 10, wherein the medicine comprises human PTH(1-84) as an active ingredient.

12. The medicine for use according to claim 9 or 10, wherein the medicine comprises human PTH(1-34) as an active ingredient.

13. The medicine for use according to claim 9 or 10, wherein the medicine comprises an acetate salt of human PTH(1-34) as an active ingredient.

## Patentansprüche

1. Parathyroidhormon (PTH), oder ein PTH-Derivat, das aus der Gruppe ausgewählt ist, die aus humanem PTH (1-34) und einem Acetatsalz von humanem PTH (1-34) besteht, zur Verwendung bei der Prävention und / oder der Behandlung und / oder zur Begrenzung der Verschlimmerung der Kniegelenkarthrose eines Menschen.

2. Das PTH oder PTH-Derivat zur Verwendung wie in Anspruch 1, wobei das PTH humanes PTH (1-84) ist.

3. Das PTH oder PTH-Derivat zur Verwendung wie in Anspruch 1, wobei das PTH-Derivat humanes PTH (1-34) ist.

4. Das PTH oder PTH-Derivat zur Verwendung wie in Anspruch 1, wobei das PTH-Derivat ein Acetatsalz von humanem PTH (1-34) ist.

5. Parathyroidhormon (PTH), oder ein PTH-Derivat, das aus der Gruppe ausgewählt ist, die aus humanem PTH (1-34) und einem Acetatsalz von humanem PTH (1-34) besteht, zur Verwendung bei der Prävention und / oder der Behandlung und / oder der Begrenzung der Verschlimmerung der von Osteoporose begleiteten Kniegelenkarthrose eines Menschen.

6. Das PTH oder PTH-Derivat zur Verwendung wie in Anspruch 5, wobei das PTH humanes PTH (1-84) ist.

7. Das PTH oder PTH-Derivat zur Verwendung wie in Anspruch 5, wobei das PTH-Derivat humanes PTH (1-34) ist.

8. Das PTH oder PTH-Derivat zur Verwendung wie in Anspruch 5, wobei das PTH-Derivat ein Acetatsalz von humanem PTH (1-34) ist.

9. Arzneimittel, das ein Parathyroidhormon (PTH), oder ein PTH-Derivat, das aus der Gruppe ausgewählt ist, die aus humanem PTH (1-34) und einem Acetatsalz von humanem PTH (1-34) besteht, als Wirkstoff umfasst zur Verwendung bei der Prävention und / oder der Behandlung und / oder der Begrenzung der Verschlimmerung der Kniegelenkarthrose eines Menschen.

10. Arzneimittel, das ein Parathyroidhormon (PTH), oder ein PTH-Derivat, das aus der Gruppe ausgewählt ist, die aus humanem PTH (1-34) und einem Acetatsalz von humanem PTH (1-34) besteht, als Wirkstoff umfasst zur Verwendung bei der Prävention und / oder der Behandlung und / oder der Begrenzung der Verschlimmerung der von Osteoporose begleiteten Kniegelenkarthrose eines Menschen.

11. Das Arzneimittel zur Verwendung nach Anspruch 9 oder 10, wobei das Arzneimittel humanes PTH (1-84) als einen Wirkstoff umfasst.

12. Das Arzneimittel zur Verwendung nach Anspruch 9 oder 10, wobei das Arzneimittel humanes PTH (1-34) als einen Wirkstoff umfasst.

13. Das Arzneimittel zur Verwendung nach Anspruch 9 oder 10, wobei das Arzneimittel ein Acetatsalz von humanem PTH (1-34) als einen Wirkstoff umfasst.

## Revendications

1. Hormone parathyroidienne (PTH) ou dérivé de PTH choisi dans le groupe consistant en PTH (1-34) humaine et sel d'acétate de PTH (1-34) humaine pour son utilisation dans la prévention et/ou le traitement et/ou la limitation d'une exacerbation d'une arthrose du genou humain.

2. PTH ou dérivé de PTH pour son utilisation selon la revendication 1, dans lequel la PTH est la PTH (1-84) humaine.

3. PTH ou dérivé de PTH pour son utilisation selon la revendication 1, dans lequel le dérivé de PTH est la PTH (1-34) humaine.

4. PTH ou dérivé de PTH pour son utilisation selon la revendication 1, dans lequel le dérivé de PTH est un sel d'acétate de PTH (1-34) humaine.

5. Hormone parathyroidienne (PTH) ou dérivé de PTH choisi dans le groupe consistant en PTH (1-34) humaine et sel d'acétate de PTH (1-34) humaine, pour son utilisation dans la prévention et/ou le traitement et/ou la limitation d'une exacerbation d'une arthrose du genou humain concomitante à une ostéoporose.

6. PTH ou dérivé de PTH pour son utilisation selon la revendication 5, dans lequel la PTH est la PTH (1-84) humaine.

7. PTH ou dérivé de PTH pour son utilisation selon la revendication 5, dans lequel le dérivé de PTH est la PTH (1-34) humaine.

8. PTH ou dérivé de PTH pour son utilisation selon la revendication 5, dans lequel le dérivé de PTH est un sel d'acétate de PTH (1-34) humaine.

9. Médicament comprenant une hormone parathyroidienne (PTH) ou un dérivé de PTH choisi dans le groupe consistant en PTH (1-34) humaine et sel d'acétate de PTH (1-34) humaine comme ingrédient actif pour son utilisation dans la prévention et/ou le traitement et/ou la limitation d'une exacerbation d'une arthrose du genou humain.

10. Médicament comprenant une hormone parathyroidienne (PTH) ou un dérivé de PTH choisi dans le groupe consistant en PTH (1-34) humaine et sel d'acétate de PTH (1-34) humaine, comme ingrédient actif pour son utilisation dans la prévention et/ou le traitement et/ou la limitation d'une exacerbation d'une arthrose du genou humain concomitante à une ostéoporose.

11. Médicament pour son utilisation selon la revendication 9 ou 10, dans lequel le médicament comprend la PTH (1-84) humaine comme ingrédient actif.

12. Médicament pour son utilisation selon la revendication 9 ou 10, dans lequel le médicament comprend la PTH (1-34) humaine comme ingrédient actif.

13. Médicament pour son utilisation selon la revendication 9 ou 10, dans lequel le médicament comprend un sel d'acétate de PTH (1-34) humaine comme ingrédient actif.
